# EUROPEAN PATENT SPECIFICATION

(11) **EP 0 637 306 B1**
(45) Date of publication and mention of the grant of the patent: **30.05.2001**
(21) Application number: 93908559.3
(22) Date of filing: 24.03.1993
(51) Int. Cl.: C07D 401/06, A61K 31/445

(54) **4-IMIDOMETHYL-1- [2'-PHENYL-2'-OXOETHYL]- PIPERIDINES AS SEROTONIN 5HT 2-ANTAGONISTS, THEIR PREPARATION AND USE IN THERAPY**
4-IMIDOMETHYL-1-[2'-PHENYL-2-OXOETHYL]-PIPERIDINE ALS SEROTONIN-5HT2-ANTAGONISTEN, IHRE HERSTELLUNG UND THERAPEUTISCHE ANWENDUNG
4-IMIDOMETHYL-1-[ 2'-PHENYL-2'-OXOETHYL]- PIPERIDINES EN TANT QU'INHIBITEURS DES RECEPTEURS 5HT2 A LA SEROTONINE, LEUR PREPARATION ET LEURS APPLICATIONS THERAPEUTIQUES

(30) Priority: 23.04.1992 US 872566; 10.03.1993 US 22502
(43) Date of publication of application: 08.02.1995
(73) Proprietor: MERRELL PHARMACEUTICALS INC., Bridgewater, N.J. 08807-0800 (US)
(72) Inventor: NIEDUZAK, Thaddeus, R., Golf Manor, OH 45237 (US); DUDLEY, Mark, W., Somerville, OH 45064 (US); KEHNE, John, H., Cincinnati, OH 45246 (US)
(74) Representative: Minoja, Fabrizio, Dr.
(86) International application number: US9302771
(87) International publication number: WO9322309

(56) References cited:
- EP-A- 0 261 688
- EP-A- 0 463 596
- EP-A- 0 468 187
- WO-A-91/06297
- WO-A-92/18127
- JOURNAL OF MEDICINAL CHEMISTRY. vol. 35, no. 24, 27 November 1992, WASHINGTON US pages 4542 - 4548 H. SUGIMOTO ET. AL. 'Synthesis and Structure-Activity Relationships of Acetylcholinesterase Inhibitors: 1-Benzyl-4-(2-phthalimidoethyl)piperidine and Related Derivatives.'

## Description

The present invention is directed to a new class of serotonin antagonists, their use in the treatment of a number of disease states, and to pharmaceutical compositions containing them.

In accordance with the present invention, a new class of serotonin 5HT₂ antagonists have been discovered that can be represented by the following formula: 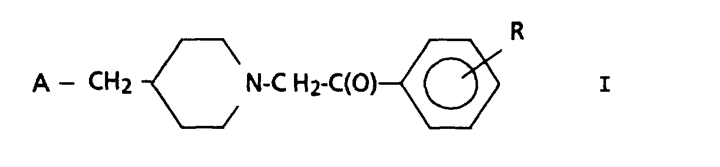 in which R is represented by hydrogen, halogen, C₁₋₄ alkyl, C₁₋₄ alkoxy, -CF₃, -OH, or -OCF₃; and A is represented by one of the following imide derivatives: 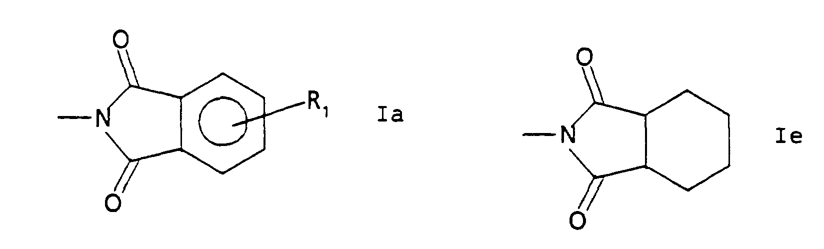 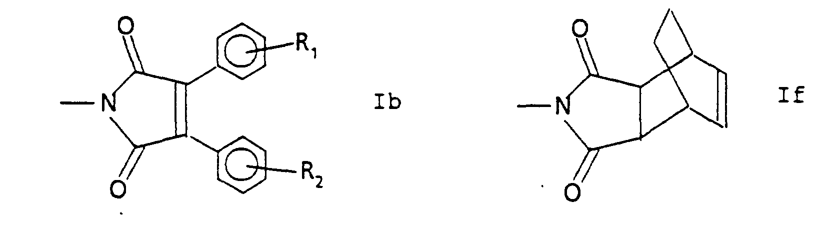 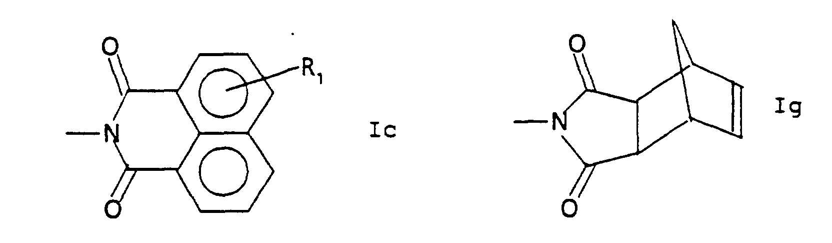 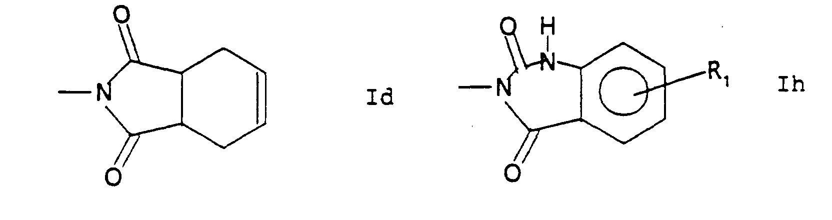 in which R₁ and R₂ are each independently represented by hydrogen, halogen, C₁₋₄ alkyl, C₁₋₄ alkoxy, -CF₃, -OH, or -OCF₃; and the pharmaceutically acceptable salts thereof.

Since the compounds of Formula I are serotonin 5HT₂ antagonists, they are effective in the treatment of a number of disease states. These disease states include anxiety, angina, anorexia nervosa, Raynaud's phenomenon, intermittent claudication, coronary or peripheral vasospasms, fibromyalgia, psychosis, drug abuse, thrombotic illness, glaucoma and in controlling the extrapyramidal symptoms associated with neuroleptic therapy.

As used in this application:
a) the term "halogen" refers to a fluorine, chlorine, or bromine atom;
b) the term "C₁₋₄ alkyl" refers to a branched or straight chained alkyl group containing from 1-4 carbon atoms, such as methyl, ethyl, n-propyl, isopropyl, n-butyl, isobutyl, etc.;
c) the term "C₁₋₄ alkoxy" refers to a straight or branched alkoxy group containing from 1-4 carbon atoms, such as methoxy, ethoxy, n-propoxy, isopropoxy, n-butoxy, isobutoxy, etc;
d) the term "C(O)" refers to a carbonyl group.

Some of the compounds of Formula I will exist as pharmaceutically acceptable basic additions salts. The expression "pharmaceutically acceptable basic addition salts" is intended to apply to any non-toxic organic or inorganic basic addition salts of the compounds represented by Formula I or any of its intermediates. Illustrative bases which form suitable salts include alkali metal or alkaline-earth metal hydroxides such as sodium, potassium, calcium, magnesium, or barium hydroxides; ammonia, and aliphatic, alicyclic, or aromatic organic amines such as methylamine, dimethylamine, trimethylamine, and picoline.

Some of the compounds of Formula I will exist as pharmaceutically acceptable acid addition salts. The expression "pharmaceutically acceptable acid addition salts" is intended to apply to any non-toxic organic or inorganic acid addition salt of the base compounds represented by Formula I or any of its intermediates. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tri-carboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicyclic, 2-phenoxybenzoic, p-toluenesulfonic acid and sulfonic acids such as methanesulfonic acid and 2-hydroxyethanesulfonic acid. Either the mono- or di-acid salts can be formed, and such salts can exist in either a hydrated or substantially anhydrous form. In general, the acid addition salts of these compounds are soluble in water and various hydrophilic organic solvents and which in comparison to their free base forms, generally demonstrate higher melting points.

As is indicated by the possible definitions for the R, the phenyl ring adjacent to the 1-position of the piperidine ring may be optionally substituted. R may represent up to 3-non-hydrogen substituents. These substituents may be located at any of the ortho, meta, or para position of the phenyl ring.

As is indicated by the definitions for A, the 4-position of the piperdine ring can be substituted with a number of imide derivatives. These various derivatives, their names and numbering are presented below to further illustrate the invention:

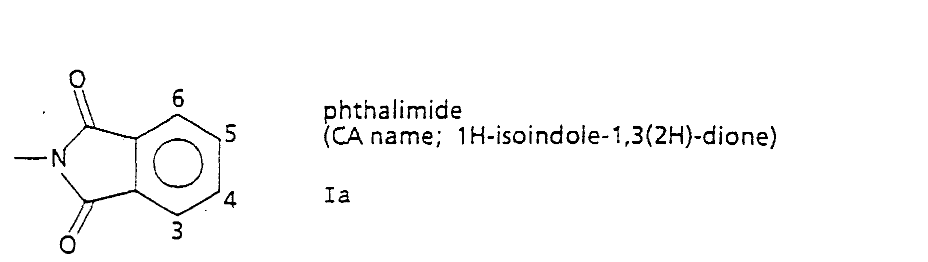

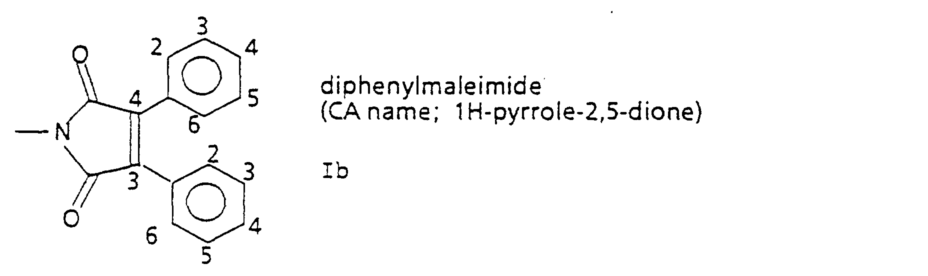

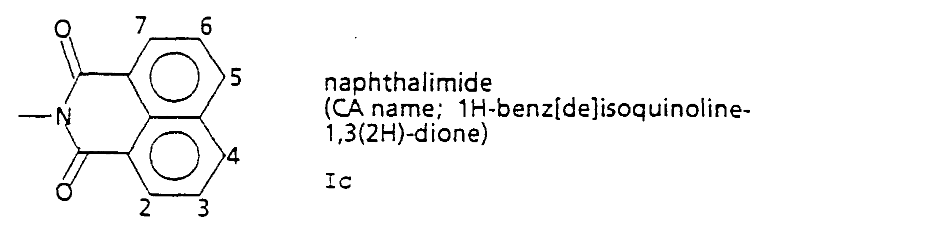

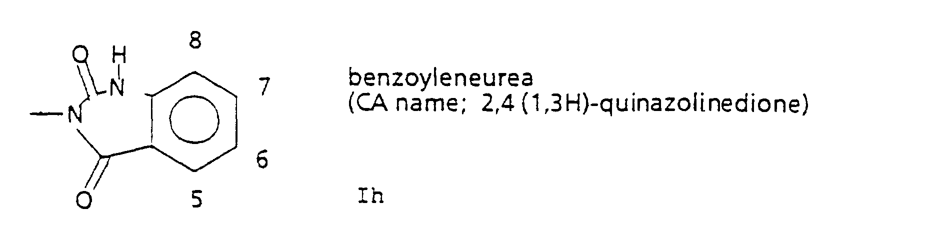

The phthalimide derivatives of Formula Ia, the diphenylmaleimide derivatives of Formula Ib, the naphthalimide derivatives of Formula Ic and the benzoyleneurea derivatives of Formula Ih may be further substituted as is depicted by the R₁ and R₂ substituents. In the phthalimide derivatives of Formula Ia, R₁ may represent up to 3 non-hydrogen substituents which may be located at any of positions 3-6 on the phthalimide structure. In the diphenylmaleimide derivative of formula Ib, R₁ and R₂ may each independently represent up to 3-nonhydrogen substituents which may be located at positions 2-6 on each phenyl. Likewise in the naphthalimide R₁ may represent up to 3 nonhydrogen substituents which may be located at positions 2-7 on this structure and in the benzoyleneurea, R₁ may represent up to 3 non-hydrogen substituents which may be located at positions 5-8 on this structure.

The cyclohexanedicarboxyimide derivatives of Formula Id and Ie will exist as configurational isomers. Any reference to these compounds should be construed as referring to either the trans isomer, the cis isomer or a mixture of these isomers. The individual configurational isomers may be obtained by use of starting materials with the desired isomer configuration.

A group of preferred compounds of Formula I includes those compounds wherein R represents a halogen substituent at the para-position.

Another group of preferred compounds of Formula I includes those compounds wherein R represents fluorine at the para-position.

Examples of compounds encompassed by Formula I include:
a) 2-[[1-[2-(4-fluorophenyl)-2-oxoethyl]-4-piperidinyl]methyl]-3a,4,7,7a-tetrahydro-4,7-ethano-1H-isoindole-1,3(2H)-dione;
b) 2-[[1-[2-(4-fluorophenyl)-2-oxoethyl]-4-piperidinyl]methyl]-1H-isoindole-1,3(2H)-dione;
c) 2-[[1-[2-(4-fluorophenyl)-2-oxoethyl]-4-piperidinyl]methyl]-3a,4,7,7a-tetrahydro-1H-isoindole-1,3(2H)-dione;
d) cis-2-[[1-[2-(4-fluorophenyl)-2-oxoethyl]-4-piperidinyl]methyl]hexahydro-1H-isoindole-1,3(2H)-dione;
e) trans-2-[[1-[2-(4-fluorophenyl)-2-oxoethyl]-4-piperidinyl]methyl]hexahydro-1H-isoindole-1,3(2H)-dione;
f) 2-[[1-[2-(4-fluorophenyl)-2-oxoethyl]-4-piperidinyl]methyl]-3a,4,7,7a-tetrahydro-4,7-methano-1H-isoindole-1,3(2H)-dione;
g) 1-[[1-[2-(4-fluorophenyl)-2-oxoethyl]-4-piperidinyl]methyl]-3,4-diphenyl-1H-pyrrole-2,5-dione;
h) 2-[[1-[2-(4-fluorophenyl)-2-oxoethyl]-4-piperidinyl]methyl]-5-methyl-1H-isoindole-1,3(2H)-dione;
i) 2-[[1-[2-(4-fluorophenyl)-2-oxoethyl]-4-piperidinyl]methyl]-1H-benz[de]isoquinoline-1,3(2H)-dione;
j) 2-[[1-[2-phenyl-2-oxoethyl]-4-piperidinyl]methyl]-1H-isoindole-1,3(2H)-dione;
k) 2-[[1-[2-(4-fluorophenyl)-2-oxoethyl]-4-piperidinyl]methyl]-4-fluoro-1H-isoindole-1,3(2H)-dione; and
1) 2-[[1-[2-(4-fluorophenyl)-2-oxoethyl]-4-piperidinyl]methyl]-benzoyleneurea.

The compounds of Formula I can be prepared utilizing synthetic methods analogously known in the art. One suitable method is depicted below in Reaction Scheme I wherein all substituents, unless otherwise indicated, are previously defined.

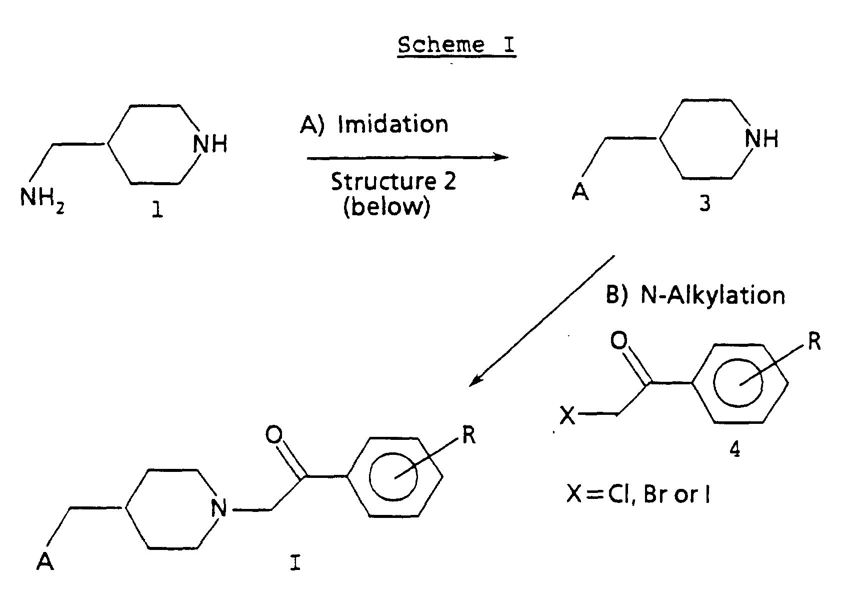

### Structure 2 is represented by one of the following:

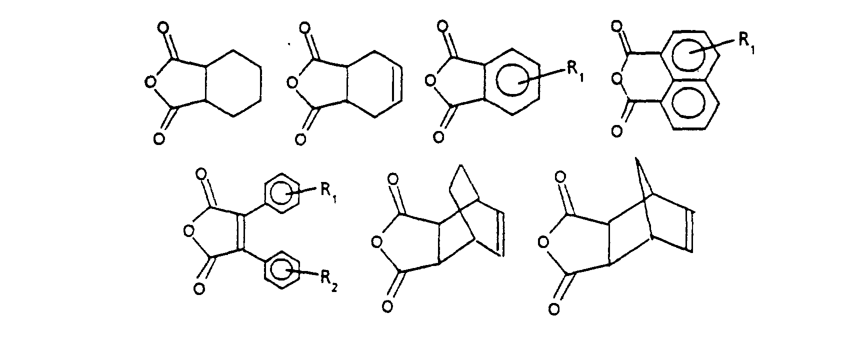

In step A, the imidation is performed by treating the appropriate cyclic anhydride defined by structure 2 with 4-(aminomethyl)piperidine of structure 1 to provide the desired cyclic imide defined by structure 3. In step B, the cyclic imide is N-alkylated with the appropriate alkyl halide of structure 4 under mild basic conditions to provide the desired compound of Formula I.

For example, in step A, the 4-(aminomethyl)piperidine of structure 1 is treated with an equivalent of the appropriately substituted cyclic anhydride defined by structure 2, such as phthalic anhydride, in a suitable organic solvent, such as xylene, or a solvent mixture, such as xylene:2-pentanone. The reaction is heated to reflux for approximately 12 to 24 hours with removal of water. The reaction is then filtered and the filtrate concentrated. The crude material can be isolated and purified using a variety of techniques known in the art, to provide the desired cyclic imide defined by structure 3.

Optionally in step A, the 4-(aminomethyl)piperidine of structure 1 can be treated with an equivalent of the appropriately substituted cyclic anhydride defined by structure 2, such as phthalic anhydride, and heated to approximately 170°C for about 1 hour. The crude material can be isolated and purified using a variety of techniques known in the art, to provide the desired cyclic imide defined by structure 3.

In step B, the cyclic imide defined by structure 3 is treated with an excess of a mild base, such as sodium bicarbonate or potassium hydrogen carbonate, in a suitable solvent mixture, such as tetrahydrofuran:water. This mixture is stirred for a short period and 1 equivalent of an appropriately substituted alkyl halide defined by structure 4, such as 2-chloro-4'-fluoroacetophenone, is added to the mixture. The reaction is then heated to reflux for approximately 2 hours. The crude material can be isolated and purified using a variety of techniques known in the art, to provide the desired product defined by Formula I.

Another suitable method to prepare the compounds of Formula I is depicted below in Scheme II wherein all substituents, unless otherwise indicated, are previously defined.

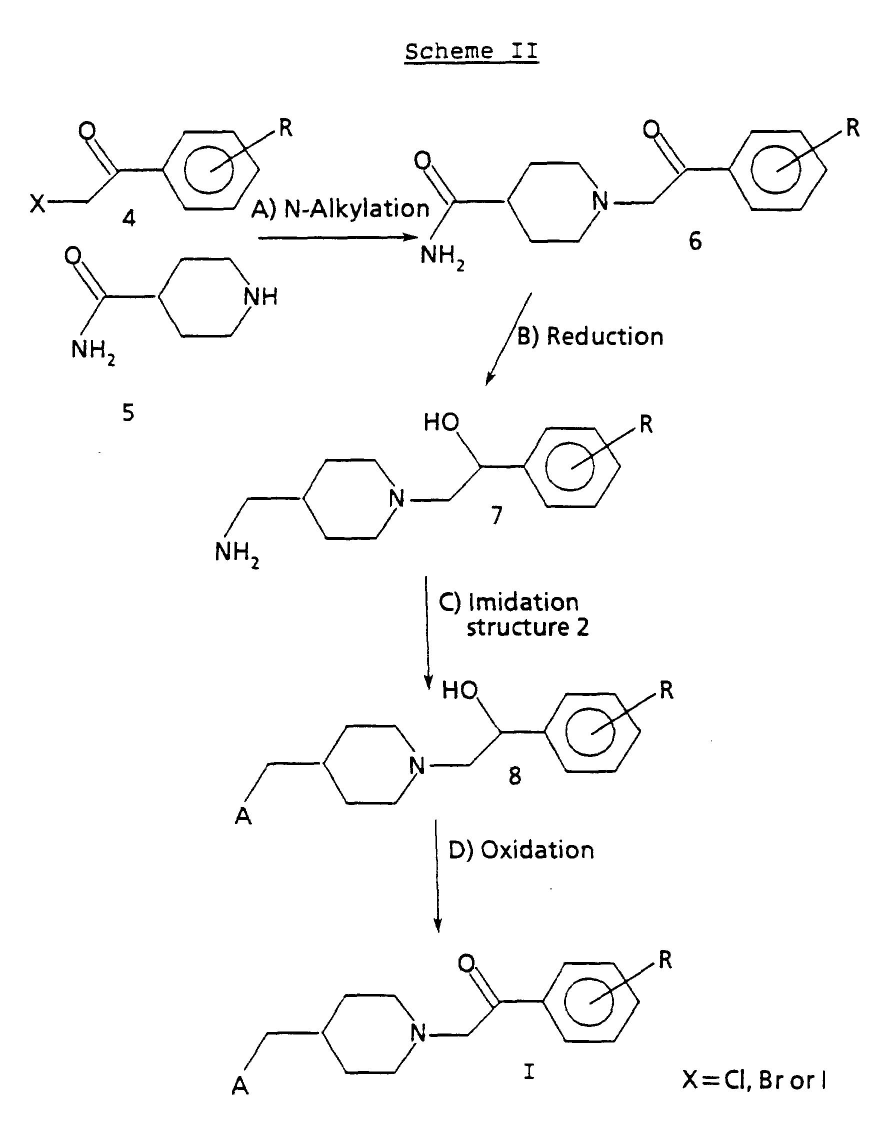

In step A, isonipecotamide of structure 5 is N-alkylated with the appropriate alkyl halide of structure 4 under mild basic conditions to produce the tertiary amine of structure 6. In step B, the carbonyl and the amide functionalities on structure 6 are reduced to the primary amine and secondary hydroxyl using a suitable reducing agent to provide the compound defined by structure 7. In step C, the imidation is performed by reacting the primary amine with the appropriately substituted cyclic anhydride defined by structure 2 in Scheme I, to provide the cyclic imide defined by structure 8. In step D, the secondary hydroxyl group is oxidized utilizing a suitable oxidizing agent to provide the desired product defined by Formula I.

For example, in step A, isonipecotamide of structure 5 is combined with an equivalent of the appropriately substituted alkyl halide of structure 4, such as 2-chloro-4'-fluoroacetophenone, in a suitable organic solvent, such as 2-propanol. The mixture is then treated with excess mild base, such a sodium bicarbonate, and the reaction is refluxed for approximately 4 hours. The reaction is then diluted with water and extracted with a suitable organic solvent such as ethyl acetate, dried over a suitable drying agent such as anhydrous magnesium sulfate, filtered and concentrated to provide the N-alkylated tertiary amine defined by structure 6.

In step B, the N-alkylated compound from above is dissolved in a suitable aprotic organic solvent, such as tetrahydrofuran, and treated with 2 equivalents of a suitable reducing agent, such as lithium aluminum hydride. The reaction is refluxed for approximately 24 hours. The crude material can be isolated and purified using a variety of techniques known in the art, to provide the desired primary amine defined by structure 7.

In step C, the primary amine from above is combined with an equivalent of the appropriate cyclic anhydride defined by structure 2 in Scheme I, in a suitable organic solvent, such as tetrahydrofuran and stirred for a short period at room temperature. The solvent is then removed and the reaction is heated to approximately 180°C under vacuum for about 1 hour. The crude material can be isolated and purified using a variety of techniques known in the art, to provide the desired cyclic imide defined by structure 8.

In step D, the cyclic imide from above is dissolved in an organic solvent mixture, such as dichloromethane:acetone and cooled to about 0°C. The solution is then treated with a suitable oxidizing agent, such as Jones Reagent [prepared according to **Fieser and Fieser** I, page 142], and allowed to stir for about 45 minutes with continued cooling. The crude material can be isolated and purified using a variety of techniques known in the art, to provide the desired product defined by Formula I.

A suitable method to prepare the compounds of Formula I wherein A is a benzoyleneurea derivative is depicted below in Scheme III wherein all substituents, unless otherwise indicated, are previously defined.

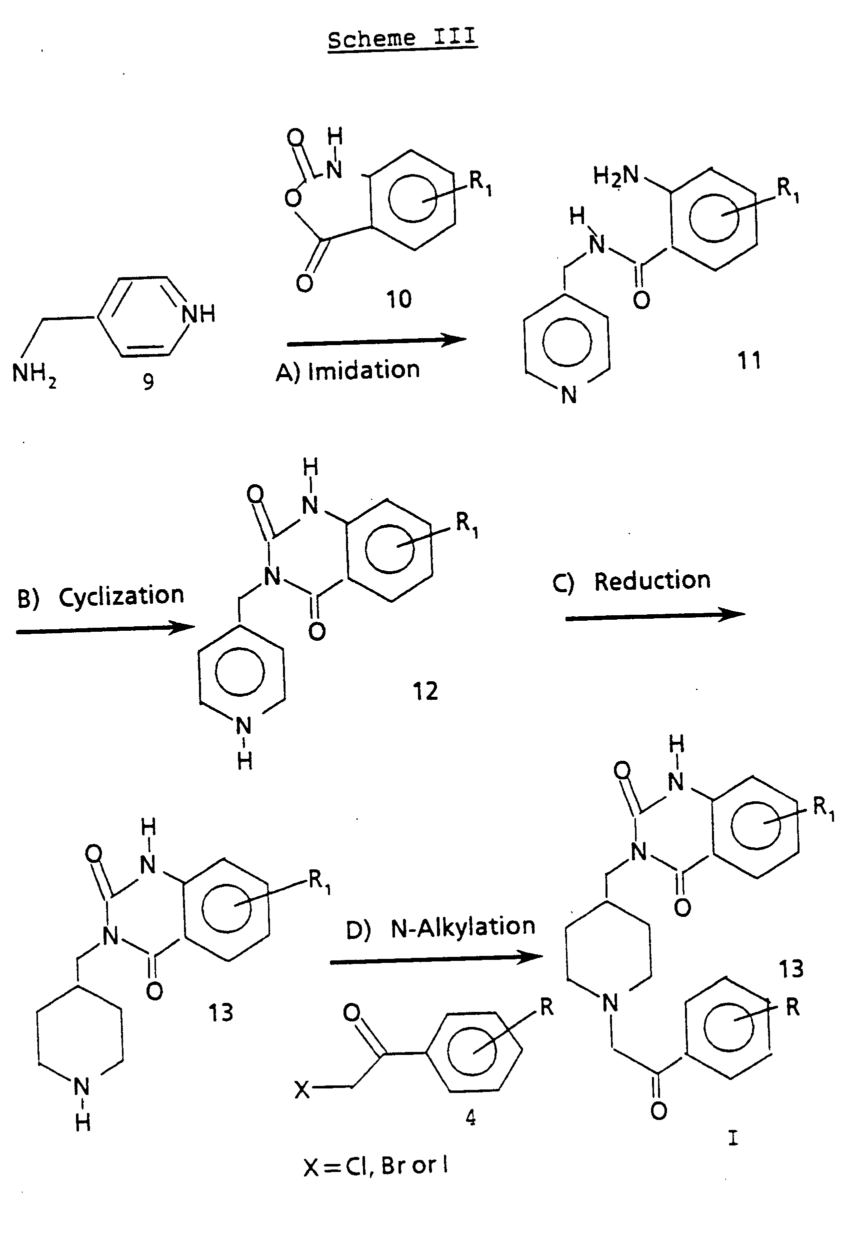

In step A, the imidation is performed by treating the appropriate cyclic anhydride defined by structure 10 with 4-(aminomethyl)pyridine of structure 9 to provide the desired amide defined by structure 11.

For example, in step A, the 4-(aminomethyl)pyridine of structure 9 is treated with an equivalent of the appropriately substituted cyclic anhydride defined by structure 10, such as isatoic anhydride, in a suitable organic solvent, such as dimethylformamide. The reaction mixture is heated to reflux for approximately 1-5 hours. The crude material can be isolated and purified using a variety of techniques known in the art, such as recrystallization, to provide the desired amide defined by structure 11.

In step B, the cyclization is performed by treating the appropriate amide defined by structure 11 with 1,1'-carbonyldiimidazole to provide the desired pyridino cyclic imide defined by structure 12.

For example, in step B, the appropriate amide defined by structure 11 is treated with an approximately equimolar amount of 1,1'-carbonyldiimidazole in a suitable organic solvent, such as tetrahydrofuran. The reaction mixture is heated under an inert atmosphere for approximately 10-40 hours. The crude material can be isolated and purified using a variety of techniques known in the art, such as recrystallization, to provide the desired pyridino cyclic imide defined by structure 12.

In step C, the reduction is performed by reducing the appropriate pyridino cyclic imide defined by structure 12 under hydrogenation conditions to provide the desired piperidino cyclic imide defined by structure 13.

For example, in step C, the appropriate pyridino cyclic imide defined by structure 12 is treated with a catalytic amount of an appropriate hydrogenation catalyst, such as PtO₂, in a suitable acidic organic solvent, such as acetic acid. The reaction mixture is then placed under a hydrogen atmosphere for approximately 5-30 hours. The reaction mixture is filtered and the filtrate concentrated. The crude material can be isolated and purified using a variety of techniques known in the art, such as recrystallization, to provide the desired piperidino cyclic imide defined by structure 13.

In step D, the N-alkylation is performed by treating the piperidino cyclic imide defined by structure 13 with an appropriately substituted alkyl halide defined by structure 4 to provide the desired compound of Formula I wherein A is benzoyleneurea derivative.

For example, in step D, the appropriate piperidino cyclic imide defined by structure 13 is treated with an excess of a mild base, such as sodium bicarbonate or potassium hydrogen carbonate, in a suitable solvent mixture, such as tetrahydrofuran:water. This mixture is stirred for a short period and 1 equivalent of an appropriately substituted alkyl halide defined by structure 4, such as 2-chloro-4'-fluoroacetophenone, is added to the mixture. The reaction is then heated to reflux for approximately 2 hours. The crude material can be isolated and purified using a variety of techniques known in the art, to provide the desired product defined by Formula I, wherein A is a benzoyleneurea derivative.

The starting materials and reagents for use in Scheme I, Scheme II and Scheme III are readily available to one of ordinary skill in the art.

The following examples presents typical syntheses as described by Scheme I, Scheme II and Scheme III. These examples are understood to be illustrative only. As used in the following examples, the following terms have the meanings indicated: "g" refers to grams, "mg" refers to milligrams, "mol" refers to moles, "mmol" refers to millimoles, "L" refers to liters, "mL" refers to milliliters, "µL" refers to microliters, "°C" refers to degrees Celsius, "TLC" refers to thin layer chromatography, "IC₅₀" refers to concentration of compound at 50% inhibition.

### Example 1

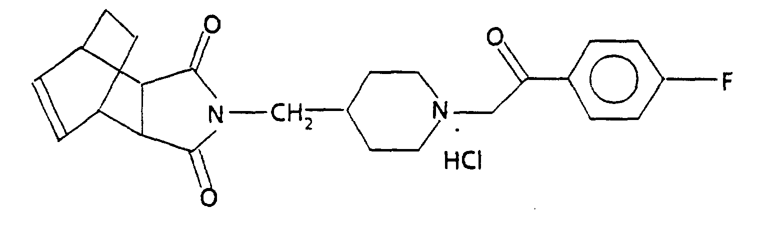

### Preparation of 2-[[1-[2-(4-fluorophenyl)-2-oxoethyl]-4-piperidinyl]methyl]-3a,4,7,7a-tetrahydro-4,7-ethano-1H-isoindole-1,3(2H)-dione monohydrochloride.

### Scheme I, Step A)

A 500 mL round bottom flask was charged with xylene (200 mL), 2-pentanone (50 mL) and endo-bicyclo[2.2.2]oct-5-ene-2,3-dicarboxylic anhydride (7.8 g, 43.8 mmol). To this was added 4-(aminomethyl)piperidine (5.0 g, 43.8 mmol). The flask was fitted with a Dean-Stark trap and heated at reflux overnight. The heat was then removed and the reaction mixture was filtered through diatomaceous earth while still hot. The solvent was then removed under vacuum. Ethyl acetate was added to the residue. Acetyl chloride (approximately 1 equivalent) and methanol were combined and added to the solution. The crude hydrochloride salt was filtered and recrystallized from methanol/ethyl acetate to provide the cyclic imide (7.5 g, 55%); mp 263-265°C.

### Scheme I, Step B)

A 500 mL round bottom flask was charged with tetrahydrofuran (150 mL), water (50 mL), sodium bicarbonate (4.05 g, 48.3 mmol) and the cyclic imide (5.0 g, 16.1 mmol) prepared above. To this was added 2-chloro-4'-fluoroacetophenone (2.8 g, 16.1 mmol). The mixture was heated to reflux for 2 hours. After cooling, saturated sodium bicarbonate was added and the reaction was extracted with ethyl acetate. The organic phase was rinsed with saturated sodium chloride, dried over anhydrous magnesium sulfate, filtered and concentrated under vacuum. The residue was dissolved in ethyl acetate. Acetyl chloride (approximately 1 equivalent) and methanol were combined and added to the solution. The crude hydrochloride salt was filtered and recrystallized from methanol/ethyl acetate to provide the title compound (4.8 g, 67%) as a white solid; mp 250°C dec.
IC₅₀=48nM (5HT₂ Binding Affinity)
Anal Calcd for C₂₄H₂₇FN₂O₃.HCl: C, 62.78; H, 6.23; N, 6.66.
Found: C, 62.80; H, 6.31; N, 6.66.

### Example 2

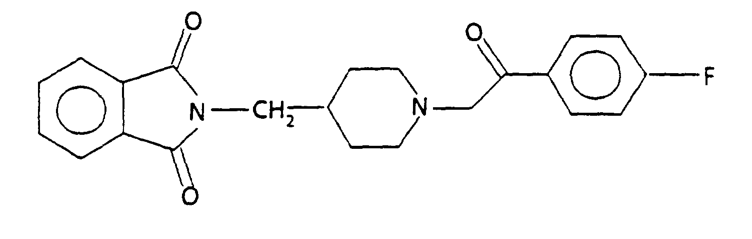

### Preparation of 2-[[1-[2-(4-fluorophenyl)-2-oxoethyl]-4-piperidinyl]methyl]-1H-isoindole-1,3(2H)-dione.

### Scheme I, Step A)

A mixture of 4-(aminomethyl)piperidine (8.0 g, 70.2 mmol) and phthalic anhydride (10.4 g, 70.2 mmol) was heated at 170°C for 1 hour. The dark, orange paste was cooled, treated with methanolic hydrogen chloride and concentrated. The crude product was recrystallized from methanol/2-butanone to provide the cyclic imide (12.0 g) as an off white powder, mp 234-237°C.

### Scheme I, Step B)

The cyclic imide prepared above (6.0g, 21.4 mmol) was combined with 2-chloro-4'-fluoroacetophenone (3.7 g, 21.4 mmol) in tetrahydrofuran (150 mL) and water (50 mL). To this was added sodium bicarbonate (5.4 g, 64.3 mmol) and the reaction was refluxed for 2 hours. After cooling, water (200 mL) was added and the reaction was extracted with ethyl acetate. The organic phase was dried over anhydrous magnesium sulfate, filtered and concentrated under vacuum. The residue was recrystallized from ethyl acetate/cyclohexane to provide the title compound (6.2 g) as a white solid, mp 110-113°C.
IC₅₀=13nM (5HT₂ Binding Affinity)
Anal Calcd for C₂₂H₂₁FN₂O₃: C, 69.46; H, 5.56; N, 7.36.
Found: C, 69.63; H, 5.60; N, 7.28.

### Example 3

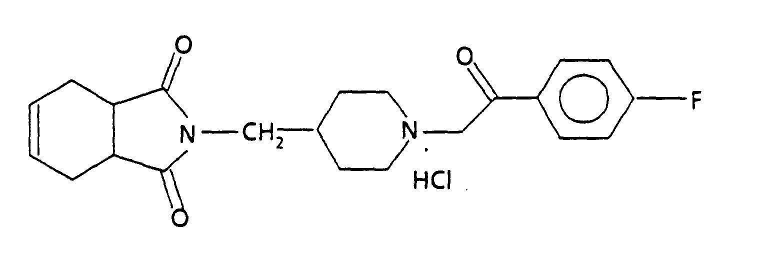

### Preparation of 2-[[1-[2-(4-fluorophenyl)-2-oxoethyl]-4-piperidinyl]methyl]-3a,4,7,7a-tetrahydro-1H-isoindole-1,3(2H)-dione monohydrohydrochloride.

### Scheme I, Step A)

In an analogous manner to Example 1, Step A, the cyclic imide ( 2.75 g, 9.7 mmol), mp 179-180°C, was prepared as the hydrochloride salt, from cis-1,2,3,6-tetrahydrophthalic anhydride ( 6 g, 39.4 mmol) and 4-(aminomethyl)piperidine (4.5 g, 39.4 mmol).

### Scheme I, Step B)

In an analogous manner to Example 1, Step B, the title compound (0.95 g, 43%) was prepared as a white solid, mp > 235°C dec, from the above cyclic imide (1.5 g, 5.3 mmol) and 2-chloro-4'-fluoroacetophenone ( 0.91 g, 5.3 mmol). IC₅₀=206nM (5HT₂ Binding Affinity)
Anal. Calcd for C₂₂H₂₅FN₂O₃·HCl: C, 62.78; H, 6.23; N, 6.66.
Found: C, 62.80; H, 6.31; N, 6.66.

### Example 4

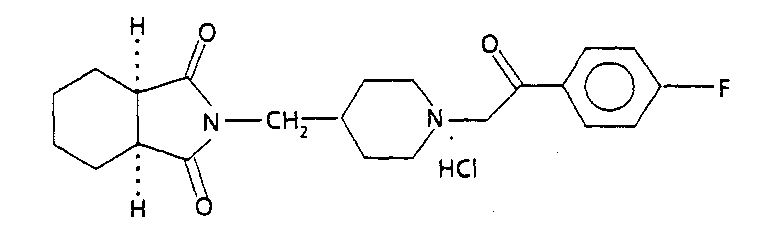

### Preparation of cis-2-[[1-[2-(4-fluorophenyl)-2-oxoethyl]-4-piperidinyl]methyl]hexahydro-1H-isoindole-1,3(2H)-dione monohydrochloride.

### Scheme I, Step A)

In an analogous manner to Example 1, Step A, the cyclic imide (9 g, 31.4 mmol), mp 148-150°C, was prepared as the hydrochloride salt, from cis-1,2-cyclohexanedicarboxylic anhydride (8.1 g, 52.5 mmol) and 4-(aminomethyl)piperidine (6.0 g, 52.5 mmol).

### Scheme I, Step B)

In an analogous manner to Example 1, Step B, the title compound (5.1 g, 69.2%) was prepared as a white solid, mp 246-248°C, from the above cyclic imide (5.0 g, 17.4 mmol) and 2-chloro-4'-fluoroacetophenone (3.01 g, 17.4 mmol). IC₅₀=162nM (5HT₂ Binding Affinity)
Anal Calcd for C₂₂H₂₇FN₂O₃·HCl: C, 62.48; H, 6.67; N, 6.62.
Found: C, 62.49; H, 6.88; N, 6.54.

### Example 5

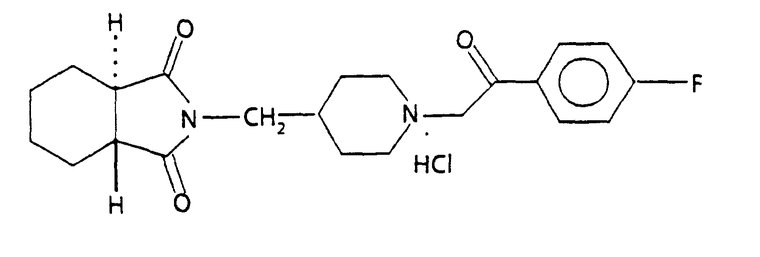

### Preparation of trans-2-[[1-[2-(4-fluorophenyl)-2-oxoethyl]-4-piperidinyl]methyl]hexahydro-1H-isoindole-1,3(2H)-dione monohydrochloride.

### Scheme I, Step A)

In an analogous manner to Example 1, Step A, the cyclic imide ( 5 g, 17.4 mmol) was prepared as the hydrochloride salt, from trans-1,2-cyclohexanedicarboxylic anhydride (8.1 g, 52.5 mmol) and 4-(aminomethyl)piperidine (6 g, 52.5 mmol).

### Scheme I, Step B)

In an analogous manner to Example 1, Step B, the title compound (5.3 g, 71.9%) was prepared as a white solid, mp 242-243°C, from the above cyclic imide (5.0 g, 17.4 mmol) and 2-chloro-4'-fluoroacetophenone (3.01 g, 17.4 mmol). IC₅₀=76nM (5HT₂ Binding Affinity)
Anal Calcd for C₂₂H₂₇FN₂O₃·HCl: C, 62.48; H, 6.67; N, 6.62.
Found: C, 62.53; H, 6.76; N, 6.64.

### Example 6

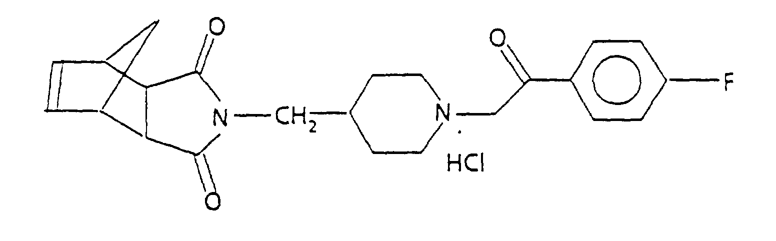

### Preparation of 2-[[1-[2-(4-fluorophenyl)-2-oxoethyl]-4-piperidinyl]methyl]-3a,4,7,7a-tetrahydro-4,7-methano-1H-isoincole-1,3(2H)-dione monohydrochloride.

### Scheme I, Step A)

In an analogous manner to Example 1, Step A, the cyclic imide ( 5 g, 16.8 mmol), mp 289°C dec., was prepared as the hydrochloride salt, from cis-5-norbornene-endo-2,3-dicarboxylic anhydride ( 7.2 g, 43.8 mmol) and 4-(aminomethyl)piperidine ( 5 g, 43.8 mmol).

### Scheme I, Step B)

In an analogous manner to Example 1, Step B, the title compound (4.0 g, 54.8%) was prepared as a white solid, mp 242-244°C, from the above cyclic imide (5.0 g, 16.8 mmol) and 2-chloro-4'-fluoroacetophenone ( 2.9 g, 16.8 mmol). IC₅₀=172nM (5HT₂ Binding Affinity)
Anal Calcd for C₂₃H₂₅FN₂O₃·HCl: C, 63.81; H, 6.05; N, 6.47.
Found: C, 63.78; H, 6.17; N, 6.06.

### Example 7

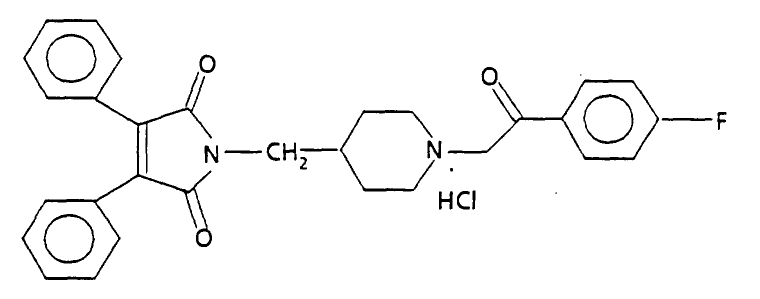

### Preparation of 1-[[1-[2-(4-fluorophenyl)-2-oxoethyl]-4-piperidinyl]methyl]-3,4-diphenyl-1H-pyrrole-2,5-dione monohydrohydrochloride.

### Scheme I, Step A)

In an analogous manner to Example 1, Step A, the cyclic imide (2.0 g, 5.2 mmol) was prepared as the hydrochloride salt, from 2,3-diphenylmaleic anhydride (5 g, 19.9 mmol) and 4-(aminomethyl)piperidine (2.3 g, 19.9 mmol).

### Scheme I, Step B)

In an analogous manner to Example 1, Step B, the title compound (1.25 g, 51%) was prepared as a light yellow solid, mp 217-218°C, from the above cyclic imide (1.8 g, 4.7 mmol) and 2-chloro-4'-fluoroacetophenone (0.81 g, 4.7 mmol). IC₅₀=307nM (5HT₂ Binding Affinity)
Anal Calcd for C₃₀H₂₇FN₂O₃·HCl: C, 69.42; H, 5.44; N, 5.40.
Found: C, 69.45; H, 5.39; N, 5.24.

### Example 8

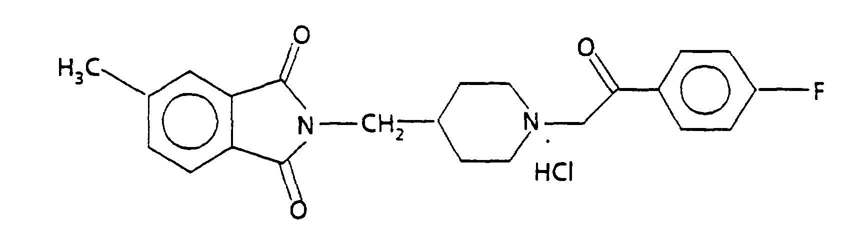

### Preparation of 2-[[1-[2-(4-fluorophenyl)-2-oxoethyl]-4-piperidinyl]methyl]-5-methyl-1H-isoindole-1,3(2H)-dione monohydrochloride.

### Scheme I, Step A)

In an analogous manner to Example 1, Step A, the cyclic imide (8.2 g, 27.8 mmol), mp 225-226°C, was prepared as the hydrochloride salt, from 4-methylphthalic anhydride (7.1 g, 43.8 mmol) and 4-(aminomethyl)piperidine (5 g, 43.8 mmol).

### Scheme I, Step B)

In an analogous manner to Example 1, Step B, the title compound (2.8 g, 38%) was prepared as an off white solid, mp 224-226°C, from the above cyclic imide (5.0 g, 16.96 mmol) and 2-chloro-4'-fluoroacetophenone ( 2.93 g, 16.96 mmol). IC₅₀=116nM (5HT₂ Binding Affinity)
Anal Calcd for C₂₃H₂₃FN₂O₃·HCl: C, 64.11; H, 5.61; N, 6.50.
Found: C, 64.25; H, 5.77; N, 6.28.

### Example 9

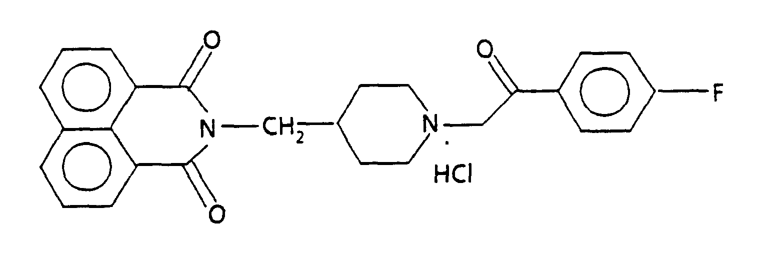

### Preparation of 2-[[1-[2-(4-fluorophenyl)-2-oxoethyl]-4-piperidinyl]methyl]-1H-benz[de]isoquinoline-1,3(2H)-dione monohydrochloride.

### Scheme I, Step A)

In an analogous manner to Example 1, Step A, the cyclic imide (3.0 g, 9.1 mmol) was prepared from 1,8-naphthalic anhydride (3.5 g, 17.5 mmol) and 4-(aminomethyl)piperidine (2.0 g, 17.5 mmol).

### Scheme I, Step B)

In an analogous manner to Example 1, Step B, the title compound (0.7 g) was prepared as a bright yellow solid, mp 260-262°C, from the above cyclic imide (1.2 g, 3.6 mmol) and 2-chloro-4'-fluoroacetophenone ( 0.69 g, 4.0 mmol). IC₅₀=96nM (5HT₂ Binding Affinity)
Anal Calcd for C₂₆H₂₃FN₂O₃.HCl: C, 66.88; H, 5.18; N, 6.00.
Found: C, 66.57; H, 5.16; N, 5.72.

### Example 10

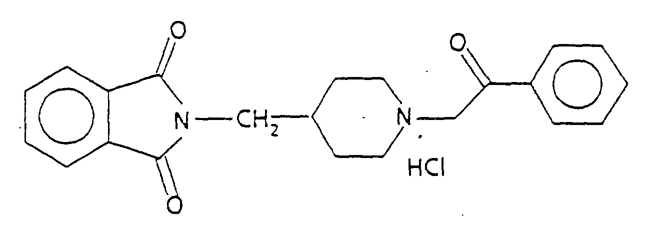

### Preparation of 2-[[1-[2-phenyl -2-oxoethyl]-4-piperidinyl]methyl]-1H-isoindole-1,3(2H)-dione monohvdrochloride.

### Scheme I, Step A)

In an analogous manner to Example 1, Step A, the cyclic imide (51.6 g, 184.3 mmol) was prepared from phthalic anhydride (50.0 g, 338.0 mmol) and 4-(aminomethyl)piperidine (38.5 g, 338.0 mmol).

### Scheme I, Step B)

In an analogous manner to Example 1, Step B, the title compound (3.70 g, 65%) was prepared as a beige solid, mp 209-211°C, from the above cyclic imide (4.0 g, 14.25 mmol) and 2-chloro-4'-fluoroacetophenone (2.98 g, 14.96 mmol). IC₅₀= 13nM (5HT₂ Binding Affinity)
Anal Calcd for C₂₂H₂₂N₂O₃.HCl: C, 66.24; H, 5.81; N, 7.02.
Found: C, 65.94; H, 6.03; N, 6.98.

### Example 11

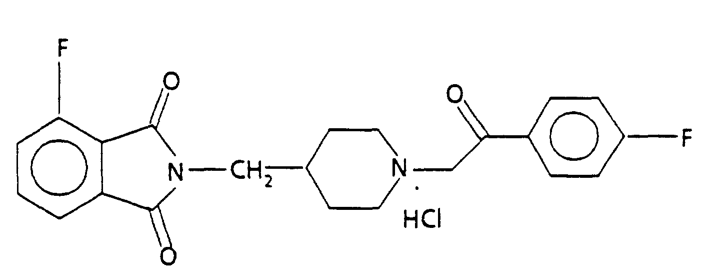

### Preparation of 2-[[1-[2-(4-fluorophenyl)-2-oxoethyl]-4-piperidinyl]methyl]-4-fluoro-1H-isoindole-1,3(2H)-dione monohydrochloride.

### Scheme II, Step A)

To a mixture of isonipecotamide (8.0 g, 62.5 mmol) and 2-chloro-4'-fluoroacetophenone (10.7 g, 62.5 mmol) in 2-propanol (300 mL) was added sodium bicarbonate (10.5 g, 125 mmol). The reaction was refluxed for 4 hours, cooled and filtered through magnesium silicate. The filtrate was concentrated under vacuum and the residue was recrystallized from ethyl acetate/methanol to provide the N-alkylated carboxamide of structure 6 (12.1 g), mp 169-172°C.

### Scheme II, Step B)

The above carboxamide (3.0 g, 11.3 mmol) was dissolved in tetrahydrofuran (150 mL) and treated with lithium aluminum hydride (0.86 g, 22.7 mmol). The reaction was refluxed for 24 hours. After cooling, the reaction was treated with water (3 mL) and 1N potassium hydroxide (5 mL) for 30 minutes. The slurry was filtered through diatomaceous earth, the filtrate was dried over anhydrous magnesium sulfate, filtered and concentrated under vacuum. The residue was converted to its p-toluenesulfonic acid salt and recrystallized from methanol/ethyl acetate to provide the primary amine of structure 7 (2.3 g), mp 195-197°C.

### Scheme II, Step C)

The above primary amine (4.8 g, 19.0 mmol) was combined with 3-fluorophthalic anhydride (3.2 g, 19.0 mmol) in tetrahydrofuran ( 200 mL) and stirred at room temperature for 3 hours. The resulting slurry was then concentrated under vacuum (1mm Eg) at 180°C for 1 hour. The reaction was cooled and the residue was recrystallized from ethyl acetate/cyclohexane to provide the cyclic imide of structure 8 as a white solid (6.7 g), mp 179-181°C.

### Scheme II, Step D)

The above cyclic imide (2.1 g, 5.2 mmol) was dissolved in a mixture of dichloromethane (60 mL) and acetone (40 mL). The solution was cooled to 0°C and treated with Jones Reagent (5 mL of a 2.6M solution prepared as described in Fieser and Fieser I, page 142). After stirring at 0°C for 45 minutes the reaction was diluted with aqueous sodium bicarbonate and extracted with dichloromethane. The organic phase was treated with methanolic hydrogen chloride and concentrated under vacuum. The resulting solid was recrystallized from ethyl acetate/methanol to provide the title compound (1.9 g) as an off white solid, mp 253-256°C. IC₅₀= 33nM (5HT₂ Binding Affinity)

### Example 12

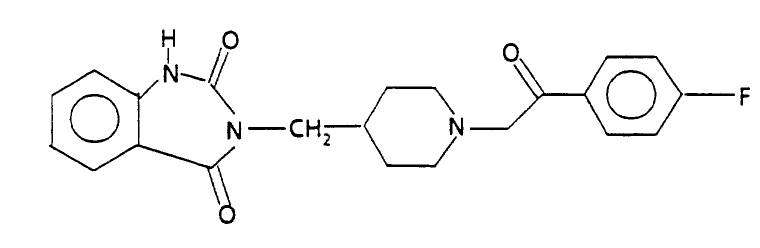

### Preparation of 2-[[1-[2-(4-fluorophenyl)-2-oxoethyl]-4-piperidinyl]methyl]-benzoyleneurea.

### Scheme III, Step A)

Dissolve 4-(aminomethyl)pyridine (15.0g, 138.7mmol) in dimethylformamide (200mL) and add isatoic anhydride (22.71g, 138.71mmol). Heat to reflux for 1.5 hours, cool to room temperature and pour into a mixture of water. Extract into ethyl acetate:toluene (2:1) and wash with water (2X), aqueous sodium hydrogen carbonate and brine (3X). Dry (MgSO₄), filter and evaporate the solvent *in vacuo* to give 25g of a beige solid. Recrystallize (2-butanone/cyclohexane) to give the amide; mp 153-155°C.
Anal. Calcd for C₁₃H₁₃N₃O: C, 68.70; H, 5.76; N, 18.49;
Found: C, 68.85; H, 5.79; N, 18.46.

### Scheme III, Step B)

Dissolve the amide prepared above (10.0g, 43.9mmol) in tetrahydrofuran (400mL) and add 1,1'-carbonyldiimidazole (7.83g, 48.28mmol). Heat to reflux under a nitrogen atmosphere overnight, cool to room temperature and add 10% aqueous hydrochloric acid (20mL). Stir for 10 minutes, add ethyl acetate and wash with aqueous sodium hydrogen carbonate then with brine. Dry (MgSO₄), filter and evaporate the solvent *in vacuo* to give 9g of a white solid. Recrystallize (2-butanone/cyclohexane) to give the pyridino cyclic imide; mp 250°C.
Anal. Calcd for C₁₄H₁₁N₃O₂: C, 66.39; H, 4.38; N, 16.59;
Found: C, 66.19, H, 4.47; N, 16.63.

### Scheme III, Step C)

Dissolve the pyridino cyclic imide prepared above (7.6g, 30.0mmol) in acetic acid (150mL) and add PtO₂ (1.5g, 6.6mmol). Place a balloon filled with H₂ on the reaction vessel and stir at room temperature and pressure for 20 hours. Remove the balloon, filter through filter aid and evaporate the solvent *in vacuo* to give a beige oil. Add ethyl acetate and filter the resulting precipitate. Recrystallize (methanol/ethyl acetate) to give the piperidino cyclic imide; mp 243-245°C.
Anal. Calcd for C₁₄H₁₇N₃O₂•C₂H₄O₂: C, 60.14; H, 6.63; N, 13.16;
Found: C, 60.30; H, 6.52; N, 13.13.

### Scheme III, Step D)

Mix the piperidino cyclic imide prepared above (4.0g, 12.5mmol), 2-chloro-4'-fluoroacetophenone (2.16g, 12.5mmol), sodium hydrogen carbonate (3.16g, 37.6mmol), tetrahydrofuran (120mL) and water (25mL). Heat at reflux for 1.5 hours, allow to cool to room temperature and add aqueous sodium hydrogen carbonate. Extract into ethyl acetate, wash with brine, dry (MgSO₄), filter and evaporate the solvent *in vacuo*. Purify by chromatography (7% methanol/chloroform) to give the title compound as a beige solid (2.5g); mp 197-200°C. IC50= 14.9nM (5HT₂ Binding Affinity)
Anal. Calcd for C₂₂H₂₂FN₃O₃ + 0.3mol H₂O: C, 65.92; H, 5.68; N, 10.48; Found: C, 65.73; H, 5.69; N, 10.37.

As noted above, the compounds of Formula I are serotonin 5HT₂ antagonists. The ability of the compounds to antagonize the effects of serotonin at the 5HT₂ receptor can be demonstrated by the spiroperidol binding test as described by Peroutka et al., in *Mol*. *Pharmacol*., Vol. 16, pages 687-699 (1979). In this test, 5HT₂ receptors are exposed to both [³H] spiroperidol, (a substance known to have a specific affinity for the receptor) and the test compound. The extent to which there is a decrease in binding of the [³H] spiroperidol to the receptor is indicative of the affinity of the test compound for the 5HT₂ receptor.

The dosage range at which the compounds exhibit their ability to block the effects of serotonin at the 5HT₂ receptor can vary depending upon the particular disease or condition being treated and its severity, the patient, other underlying disease states the patient is suffering from, and other medications that may be concurrently administered to the patient. Generally though, the compounds will exhibit their serotonin 5HT₂ antagonist properties at a dosage range of from about 0.1 mg/kg of patient body weight/day to about 100 mg/kg of patient body weight/day. The compounds are typically administered from 1-4 times daily. Alternatively, they can be administered by continuous infusion. The compounds can be administered orally or parenterally to achieve these effects.

Since the compounds are serotonin 5HT₂ antagonists, they are useful in the treatment of a variety of disease states and conditions. They are useful in the treatment of anxiety, variant angina, anorexia nervosa, Raynaud's phenomenon, intermittent claudication and coronary or peripheral vasospasms. These conditions and diseases can be relieved by administering to a patient in need thereof of, a compound of Formula I, in an amount sufficient to treat the disease or condition (i.e. an anxiolytic amount, anti-anorexic amount, anti-anginal amount, etc.). This quantity will be within the dosage range at which the compound exhibits its serotonin 5HT₂ antagonistic properties.

The compounds are also useful in the treatment of fibromyalgia. As used in this application, fibromyalgia refers to a chronic disease state wherein the patient suffers from numerous symptoms such as, for example, widespread generalized musculoskeletal pains, aching, fatigue, morning stiffness and a sleep disturbance which can be characterized as an inadequacy of stage 4 sleep. Administration of this compound, in an anti-fibromyalgia amount relieves or alleviates the symptoms the patient is experiencing. An anti-fibromyalgia amount will be within the dosage range described above wherein this compound exhibits its serotonin 5HT₂ antagonist effect.

The compounds can also be used to treat the extrapyramidal symptoms that often accompany the administration of neuroleptic agents such as haloperidol, chlorpromazine, etc. These extrapyramidal side effects (EPS) can manifest themselves in a variety of ways. Some patients experience a parkinsonian-like syndrome, wherein they experience muscular rigidity and tremors. Others experience akathisia, which can be characterized as a compelling need for the patient to be in constant movement. A few patients experience acute dystonic reactions, such as facial grimacing and torticollis. The administration of these compounds to a patient in need thereof, in an anti-EPS amount, will relieve or alleviate the symptoms that the patient is experiencing. The amount of compound which produces this anti-EPS effect is an amount within the dosage range at which this compound exhibits its serotonin 5HT₂ antagonistic effect.

As used in this application:
a) the terms "anxiety, variant angina, anorexia nervosa, Raynaud's phenomenon, and coronary vasospasms" are used in the manner defined in the 27th Edition of Dorland's Illustrated Medical Dictionary;
b) the term "patient" refers to a warm-blooded animal, such as for example rats, mice, dogs, cats, guinea pigs, and primates such as humans, and;
c) the term "treat" refers to either relieving or alleviating the patient's disease or condition.
d) any reference to "5HT₂ binding affinity" refers to the spiroperidol binding test as described by Peroutka et al., in *Mol*. *Pharmacol*., Vol. 16, pages 687-699 (1979).

The compounds of Formula I are also useful in the treatment of thrombotic illness. A thrombus is an aggregation of blood factors, primarily platelets and fibrin with entrapment of other formed elements of the blood. Thrombi can also consist of primarily platelet aggregates. Thrombi are typically formed in order to prevent excessive bleeding from injured blood vessels. Thrombi are typically formed in the following manner.

The vascular endothelium serves as a barrier between the blood-borne platelets which continually circulate throughout the body and the proaggregatory subendothelial components, which are primarily collagen. In addition to serving as a physical barrier, the cell membranes of the endothelial lining contain negatively charged components which serve to create an electrostatic repulsion between the platelets and the lining of the vessels. Trauma to the blood vessel will disrupt this endothelial lining and allow the platelets to come in contact with the underlying collagen and fibronectin. This causes the platelets to adhere to the subendothelial surface. This initial adherence causes the release, from these platelets, of a number of chemicals such as adenosine diphosphate, serotonin, and thromboxane A₂, all of which have a proaggregatory effect upon the initial platelet aggregate or plug and stimulate other circulating platelets to adhere to this newly formed plug. The additional adherence of these platelets stimulates the further release of these proaggregatory chemicals, which causes further growth of the platelet plug. Thus a self-perpetuating cycle is initiated which promotes the growth of the plug.

In addition to adhering to the injured vascular wall and forming aggregates, activated platelets accelerate the generation of thrombin which acts to convert the plasma protein, fibrinogen, into fibrin, thereby stabilizing the thrombus and promoting its growth. Prior to the conversion of fibrinogen into fibrin, a sequence of enzymatic conversions take place on the platelet surface which ultimately leads to the formation of fibrin. Both the negatively charged phospholipids on the platelet surface and calcium are essential for the maximal activation of Factor X. Once Factor X is activated, prothrombin is converted to thrombin which cleaves fibrinogen into fibrin and activates Factor XIII. This Factor catalyzes the crosslinking reaction of fibrin which stabilizes the platelet mass. In addition, thrombin is a powerful platelet activator and will act to perpetuate the process.

Thus, once the platelets come in contact with the subendothelial surface, a reaction is initiated in which a number of positive feedback control systems act to produce a thrombus which blocks off the affected vasculature. The entire process (ie. platelet aggregation, fibrin generation, and polymerization) is referred to as hemostasis and is important in the prevention of excessive bleeding from the wound.

Although the formation of thrombi is desirable in a bleeding vessel, it is pathological in an intact vessel. Thrombi occur in intact vessels due to minor alterations in the endothelial cell surface or injuries that result in the disruption of the endothelial linings. Even relatively minor alterations can allow the platelets to come in contact with collagen and initiate the process described above. These minor alterations occur from a variety of causes. These causes include stasis, (ie. decreased movement of blood in the cardiac chambers or blood vessels) which induces damage due to lack of oxygen and reduces the shear forces that ordinarily discourage platelet interaction. Another cause is the damage which the process of atherosclerosis inflicts upon the endothelial linings. Endothelial linings are known to be disrupted at the site of atherosclerotic lesion.

Thus, a significant amount of research has been focused on finding drugs which will prevent the platelets from undergoing aggregation due to these minor alterations which are commonly found on the endothelial linings. Part of the research has been directed at exploring what effect could be achieved by administering an antagonist of serotonin, one of the proaggregatory substances which is released when the platelets initially begin to aggregate. Although serotonin is a relatively weak proaggregatory factor, it has been discovered that serotonin has a synergistic effect upon the primary proaggregatory clotting factor, ADP. Thus serotonin amplifies the proaggregatory effect of ADP.

Ketanserin is a serotonin antagonist. It reacts at the 5HT₂ receptor. Bush et al. reported this compound was extremely effective in preventing thrombus formation in canine models which have been designed to screen for this activity. Drug Development Research, Vol. 7, pages 319-340 (1986).

It has been discovered that the compounds of Formula I are also effective in the prevention of acute thrombosis, especially those of the coronary arteries. The compounds decrease the rate at which platelets aggregate as the result of minor alterations in the endothelial lining of the vasculature and therefore prevent the formation of acute pathological thrombi.

Since the compounds are effective as an antithrombotic agent, they can be utilized in a variety of clinical settings in which a patient is at risk of developing pathological acute thrombi. As noted above, they should be administered on a prophylactic basis to prevent the occurrence of an acute thrombotic episode, not to lyse thrombi which have already occurred.

For example, patients who have undergone thrombolysis with agents such as tissue plasminogen activator are at a high risk of suffering subsequent acute coronary artery thrombosis. These compounds can be administered to these patients to prevent them from suffering additional acute coronary artery thrombotic episodes and any ensuing myocardial infarction.

They can also be used to decrease the time for reestablishing patent blood flow with thrombolysis, since they prevent acute thrombotic episodes. Acute thrombotic episodes routinely occur in patients undergoing thrombolysis and prolong the time required to re-establish patent blood flow. Patients who have undergone either a coronary bypass procedure or angioplasty are also typically at a greater risk of suffering thrombosis and thus can benefit from treatment as well. Other patients who will benefit from therapy include patients with saphenous vein bypass grafts, preventative therapy for acute occlusion after coronary angioplasty, secondary prevention of stroke recurrence, thrombosis of arteriovenous cannula in patients on hemodialysis and to prevent the occurrence of stroke and coronary thrombosis in patients with atrial fibrillation.

The compound can also be administered to patients to prevent the occurrence of transient ischemic attacks (TIA). These attacks result from the formation of platelet emboli in severely atherosclerotic arteries, usually one of the carotid arteries, and these attacks are the forerunners of cerebral thrombus, i.e., stroke.

Thus, the compounds can be used to prevent the occurrence of pathological acute thrombotic or embolic episodes. In order to achieve this result it is necessary that the compounds be administered to the patient in an antithrombotic quantity. The dosage range at which these compounds exhibit this antithrombotic effect can vary depending upon the severity of the thrombotic episode, the patient, other underlying disease states the patient is suffering from, and other medications that may be concurrently administered to the patient. Generally though, this compound will exhibit an antithrombotic effect at a dosage range of from about 0.1 mg/kg of patient body weight/day to about 100 mg/kg of patient body weight/day. The administration schedule will also vary widely, but will typically be from 1 to 4 times daily. This compound can be administered by a variety of routes. It is effective if administered orally or parenterally.

If desired, the compounds can be administered in combination with other antiaggretory substances, such as, for example, aspirin (300-1200mg/day), dipyridamole (300-400 mg/day), ticlopidine (50-500mg/day), warfarin (25-300 mg/day), hirudin (0.1-100 mg/kg/day), or MDL 28,050. The compound can also be administered in combination with a thromboxane synthetase inhibitor, such as, for example, ozagrel, dazmegrel, SQ 29,548, or SQ 30,741. These thromboxane synthetase inhibitors are typically administered at a dosage range of from 0.5-50mg/kg/day. The compound and the thromboxane synthetase inhibitors can be compounded into a single dosage form and administered as combination product. Methods for producing such dosage forms are well known in the art.

As used in this application, the term "antithrombotic" should be construed as referring to the ability to either prevent or decrease the formation of acute pathological thrombi or emboli. It should not be construed as referring to the ability to dissolve a thrombus that has already formed. For the purpose of this application, the difference between a thrombus and an embolus, is that an embolus can be be an entire thrombus or a portion of a thrombus, that produces occlusion by moving to the site of occlusion from other parts of the circulation. It is not produced at the site of occlusion as is a thrombus.

One of the significant problems associated with drug abuse is the high rate of relapse among patients in drug rehabilitation programs. A large percentage of patients in these programs ultimately resume their pattern of drug abuse after discharge from a rehabilitation center. It has been discovered that the compounds of Formula I can be utilized in patients recovering from drug abuse to decrease the likelihood of their relapse and readdiction to drugs. Current research indicates that these patients return to their addicted states in an attempt to return to the positive affective state produced by drug abuse (J. Stewart, et al, Psychological Reviews 91:251-268, 1984, and M.A. Bozarth and R.A. Wise, NIDA Res. Monogr. 67:190-6, 1986).

Recent research also indicates certain drugs of abuse produce this positive affective state by causing the release of dopamine in the nucleus accumbens region of the brain (meso limbic area) (Carboni, E., Acquas, E. Frau, R. & Di Chiara, G. (1989) European Journal of Pharmacology, 164, 515-519; Di Chiara, G. & Imperato, A. Journal of Pharmacology and Experimental Therapeutics, 244, 1067-1080; H.C. Fibiger et al, Annals of the New York Academy of Sciences 537:206-215, 1988 and C.J. Schmidt, et al, J. Pharmacol Exp. Ther. 256:230-235, 1991). Since nucleus accumbens dopamine release is the incentive for continued drug abuse, compounds blocking the release of dopamine and/or its physiological effects in this area of the brain would prevent the patient from receiving gratification via drug abuse. Compounds interfering with dopamine in this area of the brain could be utilized to remove the motivation to resume one's drug habits.

Schmidt et al has shown that serotonin 5HT₂ antagonists inhibit the release of dopamine in the CNS. Meert et al has shown that the 5HT₂ antagonist, ritanserin, abolished the preference for both alcohol and cocaine in a rodent model of drug abuse (T.F. Meert, et al, European Journal of Pharmacology, 183, 1924).

The compounds of Formula I are serotonin 5HT₂ antagonists. They can be utilized in the treatment of drug abuse to remove the gratification obtained from drug abuse and decrease the likelihood of readdiction. These compounds can be utilized to prevent patients from becoming readdicted to alcohol, nicotine, opiates and psychostimulants such as cocaine, amphetamine, methamphetamine, dextroamphetamine, etc.

The compounds effectiveness in treating drug abuse can be demonstrated in in-vivo animal models known in the art. One such model is the rodent self-stimulation model as described in R.A. Frank, et al, (1987) Behavioral Neuroscience, 101, 546-559. In this model, rats are implanted with bipolar stimulating electrodes in the ventral tegmental area of the brain. The rats are trained to stimulate themselves and a control current is established. This group is then given cocaine, for example, and a second level of stimulation is established. Drugs of abuse, such as cocaine, typically lower the level of current that is required for self-stimulation. The test compound is then administered in the presence of cocaine or another drug of abuse. If the compound is preventing the effects of dopamine in the mesolimbic area, then the level of current required for stimulation returns toward the control level. Other models include C. Kornetsky, et al. Testing and Evaluation of Drugs of Abuse, New York, Wiley-Liss, 1990 and J.R. Stellar, et al, The Neuropharmacological Basis of Reward, Oxford U.K., Clarendon Press, 1989.

In order to exhibit this anti-drug abuse potential, the compounds need to be administered in a quantity sufficient to inhibit the release of dopamine in the mesolimbic area of the brain. The dosage range at which these compounds exhibit this effect can vary widely depending upon the particular drug of abuse, the severity of the patient's addiction, the patient, the route of administration, and the presence of other underlying disease states within the patient, etc. Typically the compounds exhibit their effects at a dosage range of from about 0.1 mg/kg/day to about 100 mg/kg/day. Repetitive daily administration may be desirable and will vary according to the conditions outlined above. Typically, the compounds will be administered from 1-4 times daily.

As used herein "treating drug abuse" refers to the compounds ability to negate the gratification which the individual receives from abusing drugs, thereby removing the motivation to resume previous drug habits or establish new ones.

Since the compounds of Formula I inhibit the release of dopamine in the CNS, they will be effective in the treatment of psychotic illnesses such as schizophrenia, mania, etc. The dosage range at which these compounds exhibit this antipsychotic effect can vary widely depending upon the particular disease being treated, the severity of the patient's disease, the patient, the route of administration, and the presence of other underlying disease states within the patient, etc. Typically the compound exhibits its antipsychotic effects at a dosage range of from about 0.1 mg/kg/day to about 100 mg/kg/day. Repetitive daily administration may be desirable and will vary according to the conditions outlined above. Typically, the compounds will be administered from 1-4 times daily.

As used in this application:
a) the term "psychosis" refers to a condition where the patient, e.g., a human, experiences a major mental disorder of organic and/or emotional origin characterized by derangement of the personality and loss of contact with reality, often with delusions, hallucinations or illusions. Representative examples of psychotic illnesses which can be treated with the compounds of the present invention include schizophrenia, and mania.

As noted above, the compounds are useful in the treatment of variant angina. Patients suffering from variant angina experience coronary vasospasms which produce the chest pains typically associated with angina. These vasospams typically occur while the patient is at rest. Patients suffering from stable angina experience these pains in response to the increased myocardial oxygen consumption associated with exercise, emotion, etc. Patients with stable angina typically have extensive coronary atherosclerosis.

Serotonin produces a biphasic response in normal coronary vessels (ie. those without significant atherosclerotic damage). Low concentrations of serotonin produce coronary dilation, whereas higher concentrations produce constriction. Patients suffering from variant angina have an abnormal response to serotonin and experience constriction at doses much lower than normal individuals. Therefore serotonin 5HT₂ antagonists benefit these patients by blocking this abnormal response to serotonin.

McFadden et al recently reported that patients with stable angina do not show a biphasic response to serotonin. Intracoronary infusion of serotonin induced constriction of the coronary vessels in these patients at all concentrations tested. The patients also experienced anginal attacks during these infusions. New England Journal of Medicine 1991; 324:648-654. Golino et al also reported similar findings. New England Journal of Medicine 1991; 324:641-648. Golino et al reported that ketanserin, a 5HT₂ antagonist, blocked coronary vessel constriction in patients with stable angina. McFadden et al and Golino et al stated that their findings suggest that serotonin, released after the intracoronary activation of platelets, contributes to or causes myocardial ischemia in patients with coronary artery disease.

Since the compounds of Formula I are serotonin 5HT₂ antagonists, they are useful in the treatment of both variant angina, unstable angina and stable angina (angina pectoris). They can also be used to treat angina which is provoked by a thrombotic or embolic episode. The compounds of Formula I can be used on a prophylactic basis to prevent the occurrence of angina or they can be administered to a patient experiencing an anginal attack to terminate that attack. The amount of compound which produces this anti-anginal effect is an amount within the dosage range at which the compounds exhibit their serotonin 5HT₂ antagonistic effects.

Glaucoma is a disorder in which elevated intraocular pressure damages the optic nerve thereby producing blindness. There are two major types of glaucoma, chronic open-angle and acute narrow-angle.

Intraocular pressure is controlled by the dynamics of aqueous humor. The aqueous humor is derived from blood by a process of secretion and ultrafiltration in the ciliary body. Aqueous humor then passes from the posterior chamber of the eye, through the pupil to fill the anterior chamber, which is the space between the back of the cornea and the plane of the iris and pupil. The aqueous humor is reabsorbed through the trabecular meshwork, located in the angle between the cornea and the iris. The aqueous humor then enters the canal of Schlemm so that it may be drained away from the eye.

In chronic open-angle glaucoma, the most common type, a defect in aqueous humor reabsorption exists at the level of the trabecular meshwork. Intraocular pressure rises above its normal maximum of 0,028 bar (21 mm Hg) due to the presence of excess aqueous humor. In acute narrow-angle glaucoma, dilation of the iris leads to the physical blockade of the entrance to the canal of Schlemm and a resulting excess of aqueous humor.

Serotoin 5HT₂ antagonists have been shown to reduce intraocular pressures and to be useful in the treatment of glaucoma, see European Patent Application 0434 021. Since the compounds of Formula I are serotonin 5HT₂ antagonist, they will be useful in the treatment of glaucoma. The dosage range at which these compounds exhibit this effect will be within the dosage ranges described above at which they exhibit their 5HT₂ antagonistic effects.

The compounds may be administered systemically to produce this effect. The compounds can also be administered topically via ophthalmic dosage forms such as, for example, ophthalmic drops, ophthalmic ointments, and ophthalmic disks. The ophthalmic drops of the present invention should contain from 0.1-10% w/w of one of the compounds of Formula
I. Typically, it will be dissolved in a buffered, isotonic solution containing antimicrobial preservative agents. The ophthalmic ointments will also generally contain from 0.1-10% w/w of one of the compounds of Formula I admixed with a suitable base, such as white petrolatum and mineral oil, along with antimicrobial preservatives. The ophthalmic disks will typically be constructed so as to contain a core of active ingredient surrounded by a polymer matrix such as, for example, a hydrophobic ethylene/vinyl acetate copolymer. Specific methods of compounding these dosage forms, as well as appropriate ophthalmic pharmaceutical carriers are known in the art. REMINGTON PHARMACEUTICALS SCIENCES, 16th Ed. Mack Publishing Co. (1980).

Typically, the ophthalmic drops or ophthalmic ointments will be administered from 1 to 4 times daily. The ophthalmic disks will be administered weekly.

The compounds of Formula I appear to have a preferential selectivity for peripheral 5HT₂ receptors in selected species. In these species it takes significantly higher doses of compound to produce an effect in conditions involved with the central nervous system than would be predicted on the basis of the compounds affinity for the 5HT₂ receptor. In these species, the compounds can be utilized in the treatment of conditions such as preventing the formation of thrombi, treating angina or for treating glaucoma with minimal CNS side effects.

The compound can be formulated into pharmaceutical dosage forms using techniques well known in the art. For oral administration, the compound can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations. In another embodiment, the compound can be tableted with conventional tablet bases such as lactose, sucrose, and cornstarch in combination with binders, such as acacia, cornstarch, or gelatin, disintegrating agents such as potato starch or alginic acid, and a lubricant such as stearic acid or magnesium stearate. Liquid preparations are prepared by dissolving the active ingredient in an aqueous or nonaqueous pharmaceutically acceptable solvent which may also contain suspending agents, sweetening agents, flavoring agents, and preservative agents as are known in the art.

For parenteral administration, the compound or its salts may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc. as are known in the art.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Pat. Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Pat. No. 3,921,636. At least two types of release are possible in these systems. Release by diffusion occurs when the matrix is non-porous. The pharmaceutically effective compound dissolves in and diffuses through the matrix itself. Release by microporous flow occurs when the pharmaceutically effective compound is transported through a liquid phase in the pores of the matrix.

The compound may be admixed with any inert carrier and utilized in laboratory assays in order to determine the concentration of the compounds within the urine, serum, etc. of the patient as is known in the art.

## Claims

1. A compound of the formula: 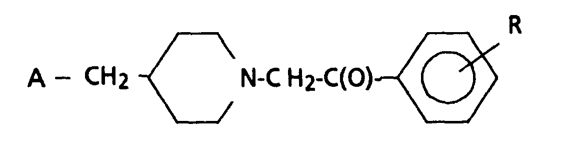 in which R represents hydrogen or from 1 to 3 substituents independently selected from halogen, C₁₋₄ alkyl,
C₁₋₄ alkoxy, -CF₃, -OH, or -OCF₃; and A represents one of the following imide derivatives: 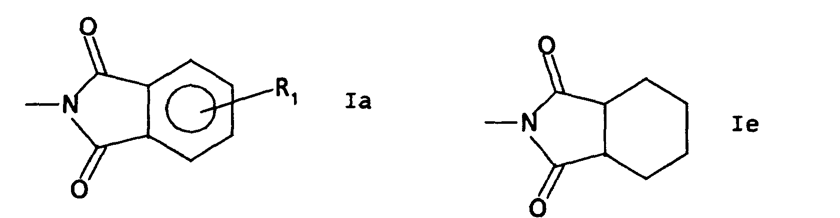 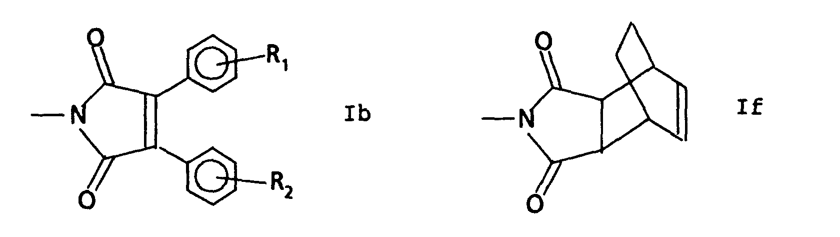 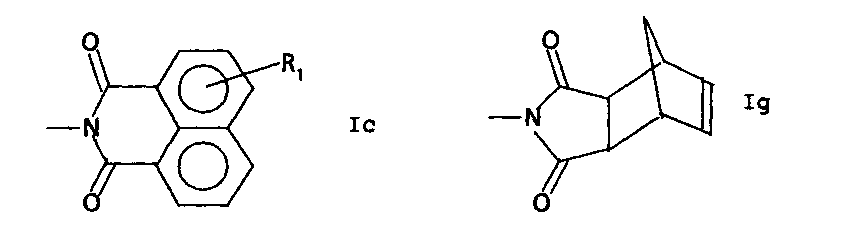 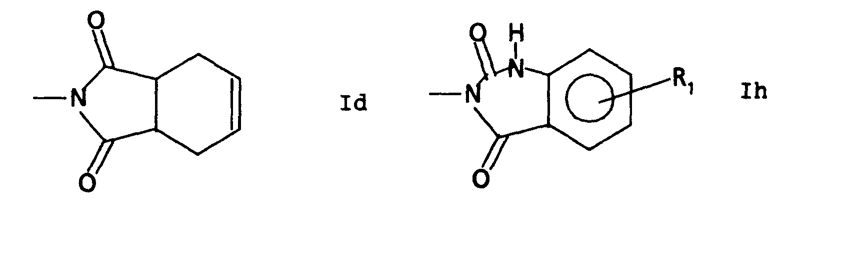 in which R₁ and R₂ are each independently represented by hydrogen, halogen, C₁₋₄ alkyl, C₁₋₄ alkoxy, -CF₃, -OH, or -OCF₃; and the pharmaceutically acceptable salts thereof.

2. A compound according to claim 1 in which R is a para-halogen substituent.

3. A compound according to claim 2 in which. R is fluorine.

4. A compound according to any one of claims 1 to 3 for use as a medicament.

5. Use of a compound according to any one of claims 1 to 3 for the preparation of a medicament for treating thrombotic illness.

6. Use of a compound according to any one of claims 1 to 3 for the preparation of a medicament for treating angina.

7. Use of a compound according to any one of claims 1 to 3 for the preparation of a medicament for treating anorexia nervosa.

8. Use of a compound according to any one of claims 1 to 3 for the preparation of a medicament for treating Raynaud's phenomenon.

9. Use of a compound according to any one of claims 1 to 3 for the preparation of a medicament for treating coronary vasospasm.

10. Use of a compound according to any one of claims 1 to 3 for the preparation of a medicament for the treatment of the extra-pyramidal side effects associated with neuroleptic therapy.

11. Use of a compound according to any one of claims 1 to 3 for preparing a medicament for relieving or alleviating anxiety.

12. A composition comprising a compound according to any one of claims 1 to 3 in admixture with an inert carrier.

13. A composition according to claim 12 wherein said inert carrier is a pharmaceutically acceptable carrier.

14. A composition according to claim 12 which additionally contains a thromboxane synthetase inhibitor.

15. A process for preparing a compound according to any one of claims 1 to 3, which comprises either of the alternate procedures (a) or (b), as follows:
(a) treating a cyclic imide of the formula: 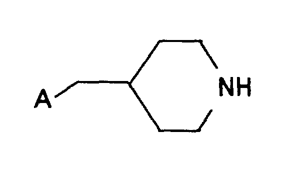 with an alkyl halide of the formula: 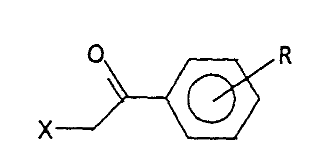 wherein X in Cl, Br or I, under mildly basic conditions;
(b) reacting a primary amine of the formula 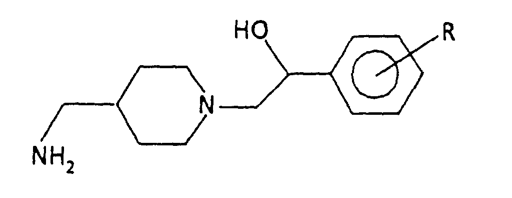 with a cyclic anhydride selected from the group consisting of 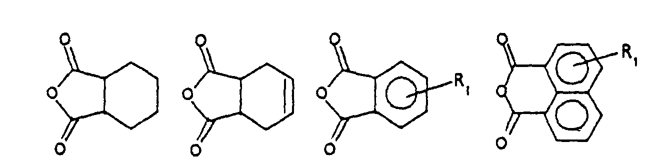 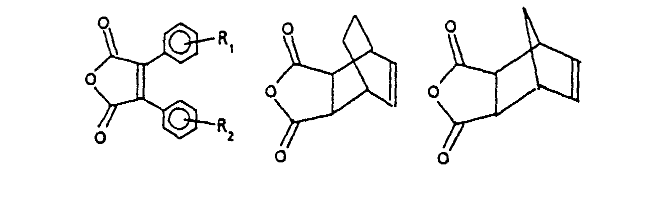 in an organic solvent while stirring at room temperature; subsequently removing the organic solvent and heating the reaction mixture for a time sufficient to form a cyclic imide of the formula: 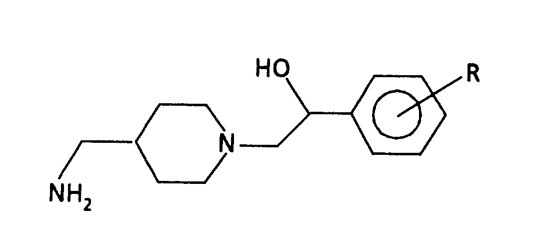 and subsequently treating this cyclic imide with an oxidizing agent in an organic solvent.

## Patentansprüche

1. Verbindung der Formel: 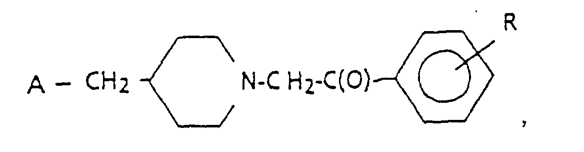 in der R ein Wasserstoffatom oder 1 bis 3 Substituenten, unabhängig voneinander ausgewählt aus einem Halogenatom, C₁₋₄-Alkyl-, C₁₋₄-Alkoxyrest, der Gruppe -CF₃, -OH oder -OCF₃, bedeutet; und A eines der folgenden Imidderivate darstellt: 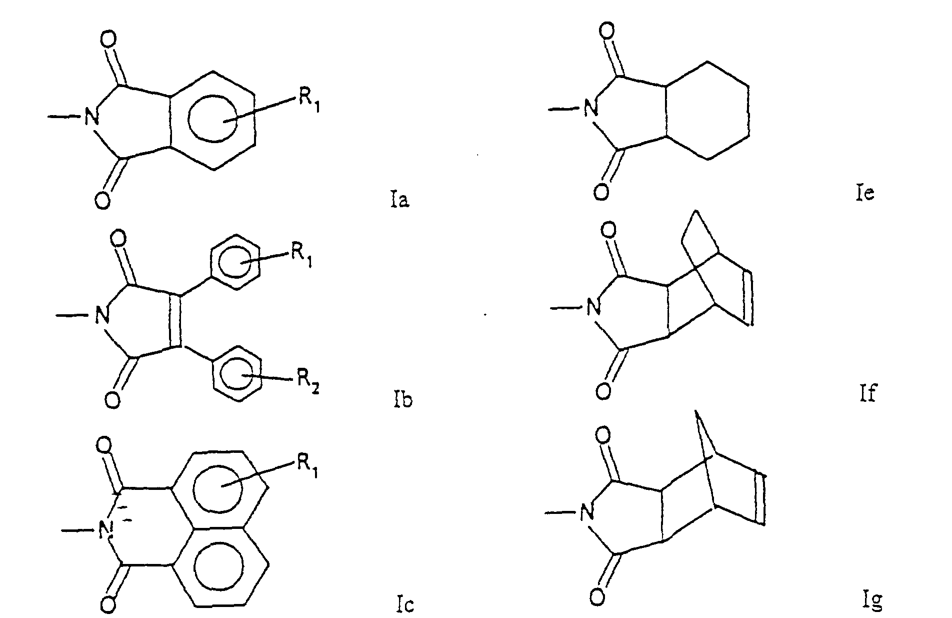 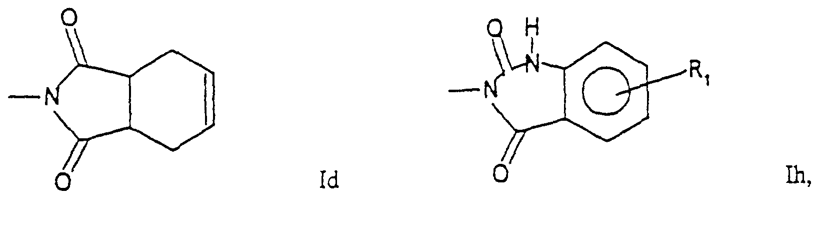 in denen R₁ und R₂ jeweils unabhängig voneinander ein Wasserstoff- oder Halogenatom, einen C₁₋₄-Alkyl-, C₁₋₄-Alkoxyrest, die Gruppe -CF₃, -OH oder -OCF₃ bedeuten; und die pharmazeutisch verträglichen Salze davon.

2. Verbindung nach Anspruch 1, wobei R einen p-Halogensubstituenten darstellt.

3. Verbindung nach Anspruch 2, wobei R ein Fluoratom bedeutet.

4. Verbindung nach einem der Ansprüche 1 bis 3 zur Verwendung als Medikament.

5. Verwendung einer Verbindung nach einem der Ansprüche 1 bis 3 zur Herstellung eines Medikaments zur Behandlung thrombotischer Erkrankungen.

6. Verwendung einer Verbindung nach einem der Ansprüche 1 bis 3 zur Herstellung eines Medikaments zur Behandlung von Angina pectoris.

7. Verwendung einer Verbindung nach einem der Ansprüche 1 bis 3 zur Herstellung eines Medikaments zur Behandlung von Anorexia nervosa.

8. Verwendung einer Verbindung nach einem der Ansprüche 1 bis 3 zur Herstellung eines Medikaments zur Behandlung des Raynaud-Phänomens.

9. Verwendung einer Verbindung nach einem der Ansprüche 1 bis 3 zur Herstellung eines Medikaments zur Behandlung von Spasmen der koronaren Gefäße.

10. Verwendung einer Verbindung nach einem der Ansprüche 1 bis 3 zur Herstellung eines Medikaments zur Behandlung von mit einer neuroleptischen Therapie verbundenen extrapyramidalen Nebenwirkungen.

11. Verwendung einer Verbindung nach einem der Ansprüche 1 bis 3 zur Herstellung eines Medikaments zur Linderung oder Milderung von Angst.

12. Mittel, umfassend eine Verbindung nach einem der Ansprüche 1 bis 3 im Gemisch mit einem inerten Träger.

13. Mittel nach Anspruch 12, wobei der inerte Träger ein pharmazeutisch verträglicher Träger ist.

14. Mittel nach Anspruch 12, das ferner einen Inhibitor der Thromboxansynthetase enthält.

15. Verfahren zur Herstellung einer Verbindung nach einem der Ansprüche 1 bis 3, das eines der alternativen Verfahren (a) oder (b) umfasst, wie folgt:
(a) Behandeln eines cyclischen Imids der Formel: 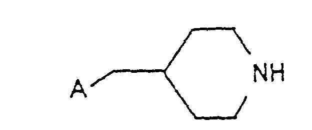 mit einem Alkylhalogenid der Formel: 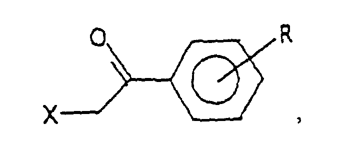 in der X ein Chlor-, Brom- oder Iodatom darstellt, unter schwach basischen Bedingungen;
(b) Umsetzen eines primären Amins der Formel: 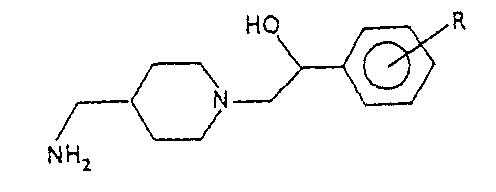 mit einem cyclischen Anhydrid, ausgewählt aus: 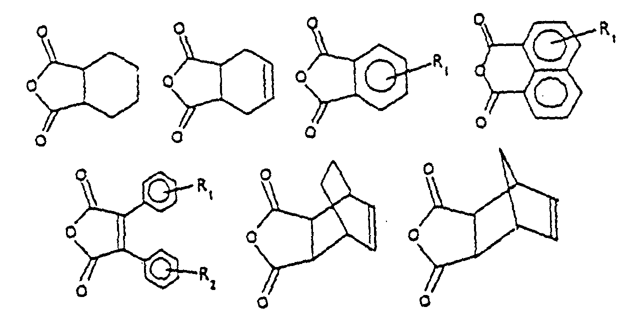 in einem organischen Lösungsmittel, während bei Raumtemperatur gerührt wird; nachfolgendes Entfernen des organischen Lösungsmittels und Erhitzen des Reaktionsgemisches für eine Zeit, die ausreicht, um ein cyclisches Imid der Formel: 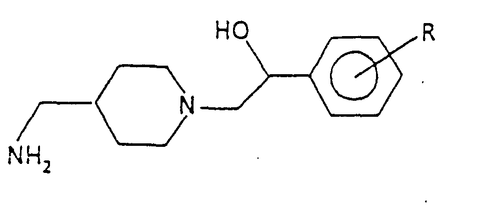 zu bilden, und nachfolgendes Behandeln dieses cyclischen Imids mit einem Oxidationsmittel in einem organischen Lösungsmittel.

## Revendications

1. Composé de formule : 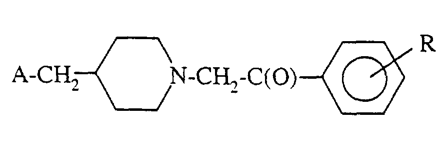 dans laquelle R représente l'hydrogène ou 1 à 3 substituants choisis indépendamment parmi halogène, alkyle en C₁₋₄, alcoxy en C₁₋₄, -CF₃, -OH et -OCF₃, et A représente l'un des dérivés imides suivants : 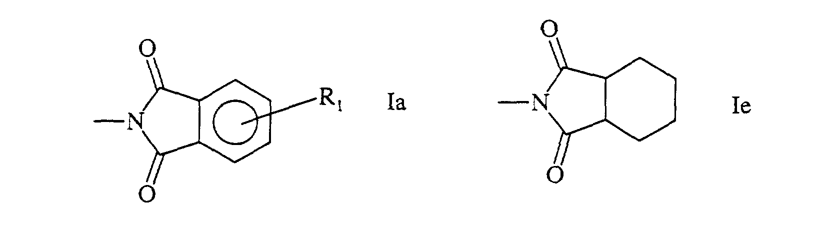 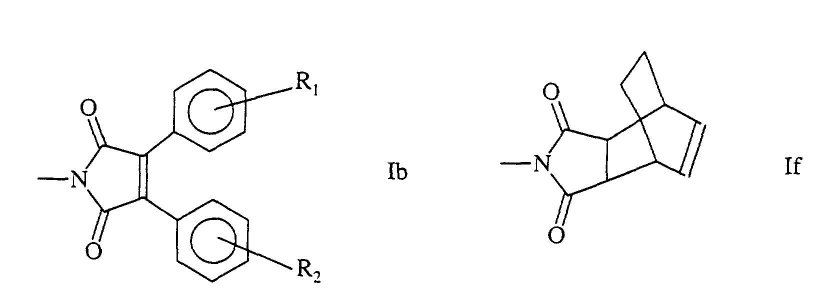 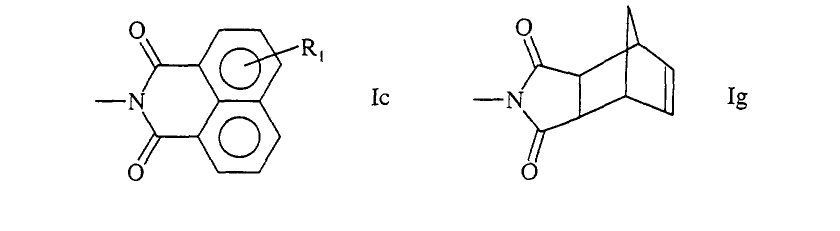 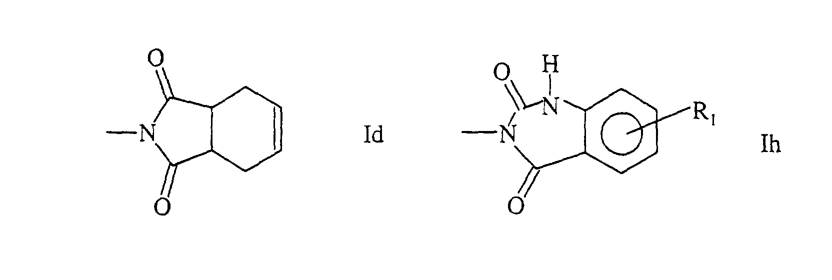 où R₁ et R₂ représentent chacun indépendamment l'hydrogène, halogène, alkyle en C₁₋₄, alcoxy en C₁₋₄, -CF₃, -OH ou -OCF₃, et ses sels pharmaceutiquement acceptables.

2. Composé selon la revendication 1, où R est un substituant halogène en para.

3. Composé selon la revendication 2, où R est le fluor.

4. Composé selon l'une quelconque des revendications 1 à 3 destiné à être utilisé comme médicament.

5. Utilisation d'un composé selon l'une quelconque des revendications 1 à 3 pour la préparation d'un médicament pour traiter la maladie thrombotique.

6. Utilisation d'un composé selon l'une quelconque des revendications 1 à 3 pour la préparation d'un médicament pour traiter l'angor.

7. Utilisation d'un composé selon l'une quelconque des revendications 1 à 3 pour la préparation d'un médicament pour traiter l'anorexie mentale.

8. Utilisation d'un composé selon l'une quelconque des revendications 1 à 3 pour la préparation d'un médicament pour traiter le phénomène de Raynaud.

9. Utilisation d'un composé selon l'une quelconque des revendications 1 à 3 pour la préparation d'un médicament pour traiter un vasospasme coronarien.

10. Utilisation d'un composé selon l'une quelconque des revendications 1 à 3 pour la préparation d'un médicament pour le traitement des effets secondaires extrapyramidaux associés à une thérapie avec des neuroleptiques.

11. Utilisation d'un composé selon l'une quelconque des revendications 1 à 3 pour la préparation d'un médicament pour calmer ou atténuer l'angoisse.

12. Composition comprenant un composé selon l'une quelconque des revendications 1 à 3 en mélange avec un support inerte.

13. Composition selon la revendication 12 où ledit support inerte est un support pharmaceutiquement acceptable.

14. Composition selon la revendication 12 qui contient en outre un inhibiteur de la thromboxane synthétase.

15. Procédé pour préparer un composé selon l'une quelconque des revendications 1 à 3, qui comprend l'un ou l'autre des protocoles (a) et (b) suivants :
(a) traiter un imide cyclique de formule : 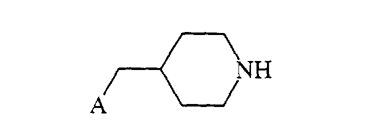 avec un halogénure d'alkyle de formule : 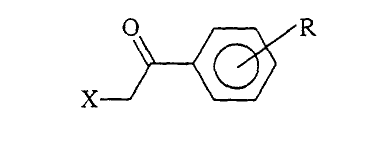 où X est Cl, Br ou I, dans des conditions basiques douces ;
(b) faire réagir une amine primaire de formule 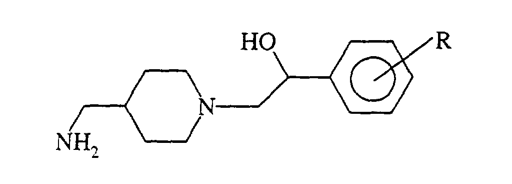 avec un anhydride cyclique choisi dans le groupe consistant en 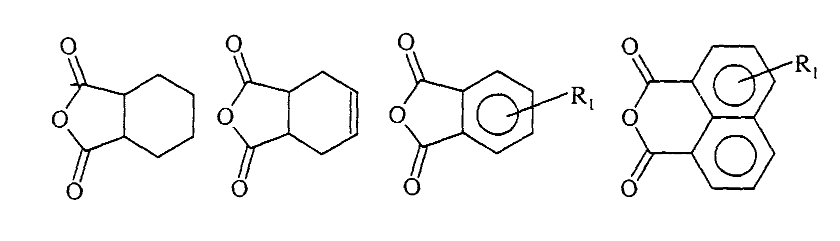 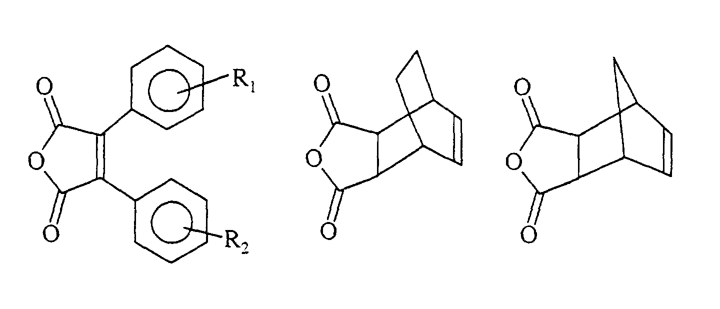 dans un solvant organique tout en agitant à la température ambiante ; puis retirer le solvant organique et chauffer le mélange réactionnel pendant une durée suffisante pour former un imide cyclique de formule 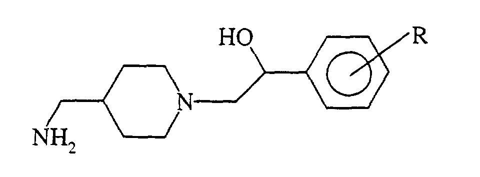 puis traiter cet imide cyclique avec un agent oxydant dans un solvant organique.
